# EUROPÄISCHE PATENTANMELDUNG

(11) **EP 0 686 400 A1**
(43) Veröffentlichungstag der Anmeldung: **13.12.1995**
(21) Anmeldenummer: 94108976.5
(22) Anmeldetag: 10.06.1994
(51) Int. Cl.: A61L 2/00, A61L 2/06, B01J 3/00

(54) **Dampfsterilisator mit Wärmerückgewinnung**

(71) Anmelder: C. STIEFENHOFER GmbH, D-86971 Peiting (DE)
(72) Erfinder: Kommermeir, Bernhard Dr., D-82396 Fischen/Ammersee (DE)

(57) **Zusammenfassung**

Die Erfindung betrifft einen Dampfsterilisator, bei dem der Dampf aus der Sterilisationskammer durch eine Dampfstrahlvakuumpumpe abgesaugt wird, und die Mischung aus abgesaugtem Dampf und Treibdampf als Heizmedium in einem Wärmetauscher genutzt wird.

## Beschreibung

Verfahrenstechnisch bedingt muß während eines Dampfsterilisationsprozesses mehrfach Dampf in die Behandlungskammer einströmen und wieder vollständig oder teilweise abgesaugt werden.
Zum einen ist dies in dem Vorphase des Sterilisationsprozesses notwendig, um aus schwer zu durchdringenden Behandlungsobjekten, z.B. Wäschepakete, die Luft nach und nach durch den Dampf auszutreiben; dieser Vorgang wird Fraktionierung genannt. Zum anderen muß nach der Sterilisations-Haltezeit das beim Aufheizen entstandene Kondensat durch Vakuumsaugen wieder verdampft und aus der Kammer abgesaugt werden.

Aktueller Stand der Technik (DIN 58946 bzw. DIN 58950) ist es, durch Einsatz eines wassergekühlten Wärmetauschers (Dampfkondensator) in Verbindung mit einer nachgeschalteten Wasserringvakuumpumpe den aus der Kammer abströmenden Dampf bei niedrigen Temperaturen zu kondensieren und dadurch das gewünschte Vakuum zu erzeugen.
Die Wasserringvakuumpumpe dient in erster Linie zur Absaugung von Inertgasen und zur Absaugung der Luft zu Beginn des Sterilisationsprozesses.

Bei einzelnen Ausführungen wird ohne Wärmetauscher gearbeitet. In diesem Fall erfolgt die Dampfkondensation innerhalb der Wasserringvakuumpumpe durch Vermischen mit dem stetig durchlaufenden Kühlwasser.

Nachteilig bei dieser Verfahrensweise ist, daß einerseits der hohe Energieinhalt des abgesaugten Dampfes ungenutzt bleibt, und andererseits zur Abfuhr dieser Wärmeenergie "verlorenes" Kühlwasser eingesetzt werden muß.

Um für den Betrieb des Dampfkondensators nicht teueres Frischwasser einzusetzen, wird dieser gelegentlich an einen bauseits vorhandenen Kühlkreislauf angeschlossen, bei dem die Wärme z.B. über einen Kühlturm abgeführt wird.

Es ist technisch problematisch und üblicherweise unwirtschaftlich die vom Dampfkondensator abgeführte Wärme zurückzugewinnen und z.B. für die bauseitige Warmwasseraufbereitung zu nutzen, da das Temperaturniveau des rücklaufenden Kühlwassers zu niedrig liegt. Dies ist dadurch bedingt, daß der weit aus größte Anteil des Dampfes unter Vakuum aus der Sterilisationskammer abgesaugt werden muß. Das Endvakuum liegt bei bis zu 50 mbar (absolut).
Aufgrund der Temperatur-Druck-Zusammenhänge des Dampfes ist die Kondensation des abgesaugten Dampfes im Wärmetauscher nur möglich, wenn die Temperatur des Kühlwassers unter der Sattdampftemperatur liegt.

Apparatetechnisch bedingt kann die Ablauftemperatur des Kühlwassers nur bei maximal 60°C ,beim Endvakuum sogar nur bei ca. 30°C liegen.
Zur Nutzung dieser Wärme müssten klassische Wärmepumpen eingesetzt werden, deren betriebswirtschaftliche Rentabilität häufig nicht gegeben ist.

***Der Erfindung liegt die Aufgabe zugrunde, die Wärmeenergie des aus der Sterilisationskammer abgesaugten Dampfes durch ein kostengünstiges System in der Art zurückzugewinnen, daß sie für nachfolgende Wärmetauschvorgänge auf einem möglichst hohen Temperaturniveau zur Verfügung steht, und gleichzeitig der Einsatz von wärmetechnisch nicht weiter nutzbarem Kühlwasser zur Dampfkondensation entfällt.***

Diese Aufgabe wird erfindungsgemäß dadurch gelöst, daß zur Absaugung von Luft und Dampf aus der Sterilisationskammer eine durch Frischdampf (Treibdampf) angetriebene Dampfstrahlvakuumpumpe eingesetzt wird und die Mischung aus Treibdampf und abgesaugtem Dampf einem Wärmetauscher zugeführt wird, über den z.B. eine gebäudetechnische Einrichtung beheizt wird. Die Dampfstrahlvakuumpumpe Kann ein- oder mehrstufig ausgeführt sein.

Das System muß so aufeinander abgestimmt sein, daß zwischen Treibdampfseite und Gemischstromseite eine ausreichende Druckdifferenz zum Betrieb der Dampfstrahlvakuumpumpe herrscht. Es ist vorteilhaft, den Druck auf der Gemischstromseite auf annähernd Atmosphärendruck einzustellen. Aufgrund der physikalischen Eigenschaften des Dampfes ergibt sich dabei eine Temperatur auf der Primärseite des Wärmetauschers von ca. 100°C (abhängig von den Mischungsverhältnissen Treibdampf/Ansaugdampf).

Dieses Temperaturniveau ist ausreichend hoch, um über den Wärmetauscher z.B. die bauseitige Warmwasseraufbereitung, Gebäudeheizung o.ä. zu unterstützen.

Durch dieses System kann die Wärmeenergie des abgesaugten Dampfes nahezu vollständig zurückgewonnen werden. Die Tatsache, daß zusätzlich Treibdampf verbraucht wird ist nicht von Schaden, da auch dessen Wärmeenergie wiedergewonnen wird.

Ein zusätzlicher Vorteil des Systems besteht darin, daß sowohl das Kondensat des Dampfes, der für den Sterilisationsprozeß eingesetzt wurde, als auch das aus dem Treibdampf vollständig zurückgewonnen werden kann, und somit der Verbrauch an frischem Kesselspeisewasser reduziert wird.

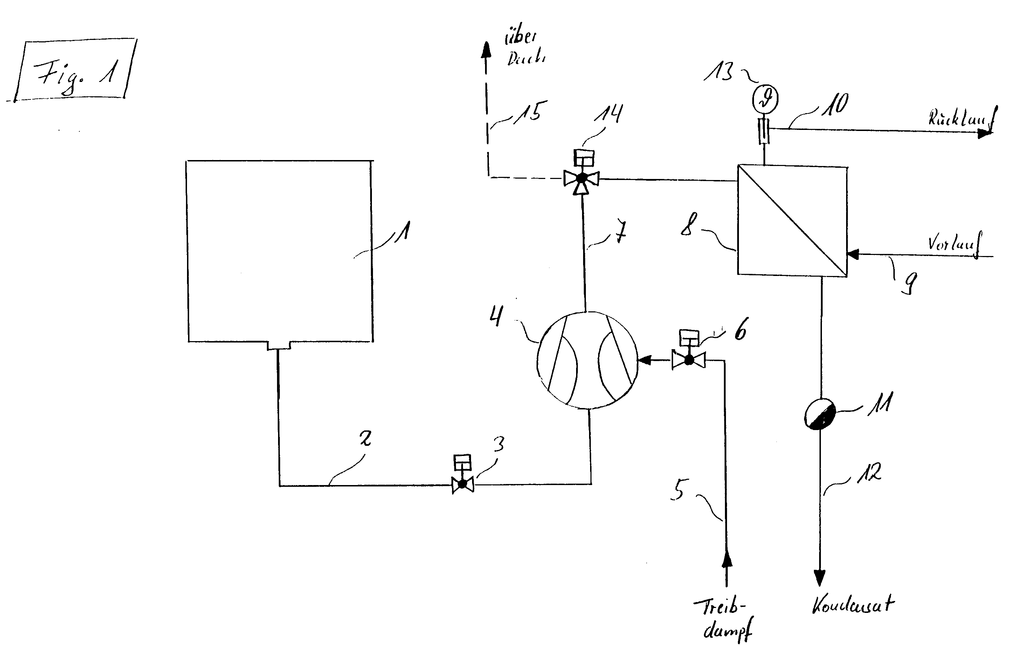

### Fig. 1 zeigt ein Funktionsschema der Erfindung.

Der Dampf wird aus dar Sterilisationskammer 1 über die Absaugleitung 2 und das Absperrventil 3 zum Ansaugstutzen der Dampfstrahlvakuumpumpe 4 geführt. Über die Dampfleitung 5 und das Absperrventil 6 strömt der Treibdampf zu. Der Gemischstrom wird über die Leitung 7 zur Primärseite das Wärmeaustauschers 8 geführt.
Die Kondensationswärme des Dampfes wird durch den Wärmeträger (z.B. Wasser), der über die Vorlaufleitung 9 zur Sekundärseite des Wärmetauschers zu- und über die Rücklaufleitung 10 abfließt, abgeführt.
Das durch Wärmeabgabe des Gemischstroms anfallende Kondensat und evtl. Inertgase werden über den Kondensatableiter 11 in die Kondensatleitung 12 abgegeben und vorzugsweise dar Dampfkesselspeisung wieder zugeführt Der Kondensatableiter 11 ist so gewählt, daß er auch zur Abfuhr von relativ großen Mengen an Inertgasen im Gemischstrom geeignet ist.

Das Absperrventil 6, Treibdampf, muß erst geöffnet werden, wenn der Druck in der Sterilisierkammer nur noch geringfügig über dem Druck im Wärmetauscher liegt (zuvor strömt der Dampf auch ohne Saugwirkung an der Dampfstrahlvakuumpumpe zum Wärmetauscher).

Durch Einsatz eines Temperaturfühlers 13 in der Rücklaufleitung 10 kann die Rücklauftemperatur geregelt werden; bei zu niedriger Temperatur kann das Treibdampfventil 6 angesteuert werden, auch wenn kein Vakuum gesaugt werden muß; bei zu hoher Temperatur kann der Dampf-Gemischstrom durch Umschalten am 3-Wegeventil 14 über die Abblaseleitung 15 "über Dach" abgelassen werden.
Die Differenz zwischen zulässiger minimaler und maximaler Rücklauftemperatur sollte möglichst groß sein, damit wegen des diskontinuierlichen Wärmeanfalls aus dem Sterilisator möglichst wenig Zusatzdampf gegeben bzw. abgeblasen werden muß (Verbraucher darauf abstimmen).

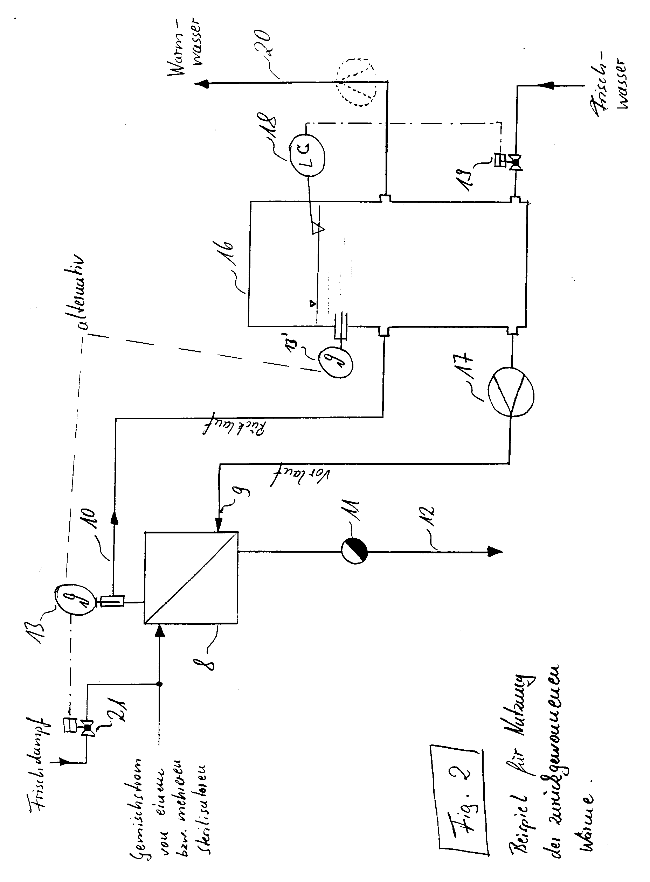

### Fig. 2 zeigt ein einfaches Beispiel zur Nutzung der zurückgewonnenen Wärme für die Warmwasserbereitung.

Die mit gleicher Nummer bezeichneten Bauteile entsprechen denen in Fig. 1.

Der Wärmetauscher kann speziell einem Sterilisator zugeordnet sein, oder zentral den Gemischstrom der Dampfstrahlvakuumpumpen von mehreren Sterilisatoren über eine Sammelleitung zugeführt bekommen.

Die aus dem Wärmetauscher abgeführte Wärme gelangt über die Rücklaufleitung 10 zum Warmwasserspeicherbehälter 16 (z.B. Drucktank mit Luftdruckpolter für entsprechenden Leitungsdruck; alternativ drucklos mit Pumpe in Warmwasserleitung 20). Über die Pumpe 17 wird das Wasser im Kreislauf gefördert.

Das Volumen des Warmwasserspeicherbehälters wird so gewählt, daß die diskontinuierlich anfallende Wärme aus dem bzw. den Sterilisatoren keine unzulässig hohen Schwankungen der Temperatur im Behälter verursacht (Pufferwirkung).
Die Anordnung des Temperaturfühlers ist auch in Behälter möglich (Position 13').

Die Nachregelung der Rücklauftemperatur über den Temperaturfühler 13 bzw. 13' kann geschehen, wie oben beschrieben, oder über ein zusätzliches Frischdampfventil 21.

Bei Wassermangel im Warmwasserspeicherbehälter spricht der Niveauregler 18 an und das Frischwasserventil 19 wird geöffnet, bis das Sollniveau wieder erreicht ist.

## Patentansprüche

1. Dampfsterilisator mit Wärmerückgewinnungseinrichtung, dadurch gekennzeichnet, daß die Sterilisationskammer über eine Dampfstrahlvakuumpumpe abgesaugt wird, und die Wärmeenergie des Gemisches aus Treibdampf und Ansaugstrom mittels Wärmetauscher genutzt wird.

2. Dampfsterilisator nach Anspruch 1, dadurch gekennzeichnet, daß bei mangelnder Wärmeabfuhr über den Wärmetauscher der Gemischstrom auch über eine Abblaseleitung ins Freie abgeblasen werden kann.

3. Dampfsterilisator nach Anspruch 1 und 2, dadurch gekennzeichnet, daß die Ablauftemperatur der Sekundärseite des Wärmetauschers auf einen definierten Wert geregelt werden kann.

4. Dampfsterilisator nach mindestens einem der vorhergehenden Ansprüche, dadurch gekennzeichnet, daß die Gemischstromleitungen von mehreren Dampfsterilisatoren zu einer gemeinsamen Sammelleitung zusammengefaßt werden können, die zu einem zentralen Wärmetauscher mit entsprechender Kapazität führt.

5. Dampfsterilisator nach mindestens einem der vorhergehenden Ansprüche, dadurch gekennzeichnet, daß der Saugstrom der Dampfstrahlvakuumpumpe und, oder der Treibstrom über Regelventile in Abhängigkeit von der gewünschten Druckabfallgeschwindigkeit in der Sterilisierkammer oder in Abhängigkeit vom Wärmebedarf am Wärmetauscher geregelt werden können.

6. Dampfsterilisator nach mindestens einem der vorhergehenden Ansprüche, dadurch gekennzeichnet, daß die diskontinuierlich anfallende Wärmeenergie durch einen Pufferspeicher auf gleichmäßigem Temperaturniveau nutzbar gemacht werden kann.

7. Dampfsterilisator nach mindestens einem der vorhergehenden Ansprüche, dadurch gekennzeichnet, daß das Kondensat des gesamten eingesetzten Dampfes wieder zurückgewonnen werden kann.
